# EUROPEAN PATENT SPECIFICATION

(11) **EP 0 700 298 B1**
(45) Date of publication and mention of the grant of the patent: **18.06.2003**
(21) Application number: 95914896.6
(22) Date of filing: 27.03.1995
(51) Int. Cl.: A61K 35/14, A61P 7/00, A61P 9/00

(54) **THERAPEUTIC USE OF HEMOGLOBIN IN THE TREATMENT OF BLOOD VESSEL BLOCKAGE**
Therapeutische Verwendung von Hämoglobin in der Behandlung einer Blutgefässblockade
UTILISATION THERAPEUTIQUE DE L'HEMOGLOBINE DANS LE TRAITEMENT DE L'OBSTRUCTION DES VAISSEAUX SANGUINS

(30) Priority: 28.03.1994 US 218536; 02.12.1994 US 350204
(43) Date of publication of application: 13.03.1996
(73) Proprietor: BAXTER INTERNATIONAL INC. (a Delaware corporation), Deerfield Illinois 60015 (US)
(72) Inventor: McKENZIE, Jack, E., McLean, VA 22101 (US); BURHOP, Kenneth, E., Mundelein, IL 60060 (US)
(74) Representative: Bassett, Richard Simon
(86) International application number: US9503788
(87) International publication number: WO95026195

(56) References cited:
- EP-A- 0 361 719
- EP-A- 0 784 983
- CHEMICAL ABSTRACTS, vol. 122, no. 13, issued 1995, March 27, (Columbus, Ohio,USA), M.P. BOWES et al. "Diaspirin cross-linked hemoglobin improves neurological outcome following reversible but not irreversible CNS ischemia in rabbits", page 45, no. 151 066q; & Stroke (Dallas) 1994, 25(11), 2253-7 (Eng).
- CHEMICAL ABSTRACTS, vol. 116, no. 13, issued 1992, March 30, (Columbus, Ohio, USA), D.M. YELLON et al.: "Myocardinal Protection: The Pathophysiology of Reperfusion and Reperfusion Injury", page 640, no. 126 261t; & Raven Press: New York, N.Y. 1991. 214 pp. (Eng).
- CHEMICAL ABSTRACTS, vol. 109, no. 21, issued 1988, November 21, (Columbus, Ohio, USA), H.W.KIM et al. "Effects of hemoglobin perfustion on contractile function of the isolated ventricular septa", page 38, no. 183 274d; & Biomater., Artif. Cells, Artif. Organs 1988, 16(1-3) 331-45 (Eng).

## Description

### Background of the Invention

The blockage of an arterial vessel produces ischemia in the tissue normally nourished by the occluded vessel. If the blockage is removed permitting reperfusion of the affected area after greater than sixty minutes of ischemia, further injury, called reperfusion injury, is paradoxically observed. This reperfusion injury is associated with a number of biochemical and physiological events such as release of intracellular enzymes, transient rise in blood pressure, reduction in contractility, influx of calcium, disruption of cell membranes, and eventual tissue necrosis (see Ferrari, et al., Am. J. Clin. Nutr. 53:2158 (1991)). It is thought that much of the tissue damage arising during ischemia and reperfusion results from the chemical action of excess amounts of accumulated oxygen free radicals (Lefer, et al., Basic Res. Cardiol., 86 Suppl. 2:109 (1991); Kirsh, et al., J. Neurotrauma, 9 Suppl. 1:S157 (1992); and Bolli, Cardiov. Drugs & Ther., 5:249 (1991)).

Experiments in a number of animal models have investigated the use of antioxidants or enzymes to control reperfusion injury. For example, Weyrich, et al., Circulation, 86:279 (1992) showed that administration of L-arginine reduced necrotic injury in a cat model of myocardial infarction. McMurray et al., J. Clin. Pharmac., 31:373 (1991) investigated sulfhydryl containing angiotensin converting enzyme inhibitors. Naslund, et al., Circ. Res., 66:1294 (1990) concluded from their work on a swine coronary model, that infarct size could be limited by administration of superoxide dismutase, but only during a very narrow window of time post-infarction. Schaer, et al., JACC, 15:1385 (1990) report a reduction in reperfusion injury by administering an acellular oxygenated perfluorochemical emulsion called Fluosol.

An important model system is percutaneous transluminal coronary angioplasty in the pig. McKenzie, et al., Cardiovascular Research, "Effects of diaspirin cross-linked hemoglobin during coronary angioplasty in the swine", 28(8):1188-1193 (1994) utilized this technique to study the effects of temporary regional myocardial ischemia. They inserted a catheter into the proximal left anterior descending coronary artery and inflated the catheter balloon to occlude the artery for a period of 4 minutes. A significant reduction in cardiac function compared to controls was observed as measured by mean arterial blood pressure (MAP), peak systolic left ventricular pressure (IVP), rate of left ventricular pressure development (dP/dt), pressure rate product (PRP), and cardiac output (CO). In addition, electrocardiograms showed elevation of the S-T segment of the ECG. These experiments are significant because McKenzie, et al. compared controls to animals receiving infusions of hemoglobin, and found that cardiac function increased significantly and the S-T segment of the ECG returned toward baseline.

The concept of infusing hemoglobin products as a substitute for blood has a long history (for a historical perspective, see R.M. Winslow, "Hemoglobin-based Red Cell Substitutes", The Johns Hopkins University Press, 1992). Free hemoglobin is not suitable for this purpose since oxygen is bound too tightly to be released in the tissues. Also, hemoglobin monomers are rapidly cleared from the blood and exhibit renal toxicity. Better success has been achieved with chemically modified hemoglobins, which assume a conformation allowing release of oxygen, and whose size and stability are more resistant to clearance.

Hemoglobins may be alpha alpha cross-linked as disclosed in U.S. Patents 4,600,531 and RE 34,271 (Walder), and virus inactivated and purified as taught in U.S. Patent 4,861,012 (Estep). Modification by pyridoxyation, carbamylation, or carboxymethylation is also known, as are chemical schemes for both cross-linking and polymerizing, as by glutaraldehyde. A summary of these chemistries is contained in Winslow, supra.

Chemical Abstracts, vol. 122(13), issued 1995, March 27 (Columbus, Ohio, USA), M.P. Bowes *et al.* describes the evaluation of the ability of diaspirin cross-linked hemoglobin (DCLHb) to reduce neurological damage in two rabbit stroke models. It was concluded that hemodilution using modified hemoglobin solutions may reduce ischemic central nervous system injury.

EP 361 719 describes a hemoglobin-based blood substitute resulting from stroma-free hemoglobin, modified with pyridoxal-5'-phosphate and intramolecularly stablised, followed by polymerisation under anaerobic conditions at an extent of 40 %. It is suggested that the blood-substitute hemoglobin-based material could be used to treat ischemic episodes including sludging and pain in sickle cell crises.

EP 784 983 discloses that extracellular hemoglobin may be used for the preparation of a medicament for the treatment of various disorders, including tissue ischemia.

### Summary of the Invention

The invention is defined in the accompanying independent claims. The medicaments that are manufactured may be used in methods for treating blockage of a blood vessel, which may be a thrombus, fat embolus, plaque, or other obstruction, or restenosis at remote time of a previously blocked vessel, which comprises administering, generally by intravenous infusion, hemoglobin to a patient undergoing tissue ischemia caused by coronary thrombosis. There are different ways of defining the therapeutically efficacious dose which may be administered.

An amount of hemoglobin may be administered which is sufficient to suppress or reduce reperfusion injury to the tissue whose nourishment has been disrupted by blockage of a blood vessel. These doses are effective not only to delimit the amount of infarcted tissue as a percentage of the cardiac tissue at risk during occlusion, but also for preventing restenosis of the vessel after the original blockage has been relieved. This protection effect is also measured by the reduction in number, magnitude, and duration to onset of ventricular arrhythmias which are known to precipitate sudden cardiac arrest in a significant proportion of patients suffering myocardial infarction. This protection is further measured by improved regional myocardial function in the border zone. Thus, the present invention affords a method for improving contractile function in ischemic cardiac tissue following relief of heart vessel blockage comprising administering hemoglobin in a dose effective to obtain a wall motion score improvement of at least 0.15 relative units between the infarct zone and 20 chords in the tissue region at risk. An effective amount is in the range of 10-2500 mg/kg of body weight, preferably 75-750 mg/kg.

The present invention thus provides a method of reducing the frequency and duration to onset of cardiac arrhythmias following relief of cardiac arterial blockage by administering hemoglobin generally in a like dose. This method also results in reducing the incidence of restenosis of a blood vessel at remote times after relief of a blockage thereof by administering hemoglobin in a like dose, which generally falls within the range of 10-2500 mg/kg of body weight, preferably 75-750 mg/kg.

The benefits and objects of administering hemoglobin as a treatment for blood vessel blockage are that it increases salvage of the area at risk, it stabilizes the circulatory system, as in cardiac ischemia, and may act directly or indirectly to lower levels of free oxygen radicals and other molecular species associated with tissue damage. It also ameliorates injury to an occluded vessel associated with restenosis of the vessel at remote times up to several weeks or longer. Many of hemoglobin's pharmacological properties are not yet understood mechanistically. It would appear that some of these properties are unrelated to oxygen-delivery since the effects are exerted at hemoglobin doses which are too low to make a significant impact on this parameter.

### Brief Description of the Drawings

Figure 1A. Effects of human serum albumin (HSA) and diaspirin cross-linked hemoglobin (DCLHb™) on the total number of reperfusion arrhythmias. The number of arrhythmias are counted from beginning of reperfusion for 45 minutes. Values are means ± SEM. *Significantly different from HSA (P<0.05).

Figure 1B. Effects of human serum albumin (HSA) and diaspirin cross-linked hemoglobin (DCLHb™) on the time to onset of reperfusion arrhythmias. The time in seconds is measured from beginning of reperfusion to the first series of reperfusion arrhythmias. Values are means ± SEM.

Figure 1C. Effects of human serum albumin (HSA) and diaspirin cross-linked hemoglobin (DCLHb™) on the total duration of reperfusion arrhythmias. The time in minutes of arrhythmias are counted from beginning of the first accelerated idioventricular beat to a time when the arrhythmias occurred less than one every 30 seconds. Values are means ± SEM. *Significantly different from HSA (P<0.10).

Figure 2. S-T Segment changes (mVolts) in HSA and DCLHb treated groups. Control (Cont) is prior to balloon occlusion. Ischemia (Isch) is 80 minutes into ischemia prior to HSA or DCLHb infusion. Reperfusion (Refer) is 3 hours into the reperfusion period.

Figure 3. Photograph comparing the infarct size in transverse cross-section between DCLHb and HSA infused test animals,

Figure 4. Effects of DCLHb and HSA on cardiac wall motion.

### Detailed Description of the Preferred Embodiment

The blockage of blood vessels may occur by any one of several mechanisms including degenerative plaque, thrombosis, fat embolus, or blood clot, and may occur in many tissues and locations of the body. The effect of such blockage is to impair or completely curtail blood flow to the portions of the vessel downstream from the blockage. The tissue nourished by the occluded vessel is thus deprived of oxygen and nutrients, and cell death may ensue. In situations where the affected vessel is a coronary artery or an artery which serves a vital brain or other organ function, the blockage may be life-threatening.

Reperfusion therapy utilizing hemoglobin is effective when some degree of blood flow is restored, or in situations in which collateral blood flow can take advantage of the increase in perfusion resulting from hemoglobin administration. Where occlusion of the blood vessel is essentially complete, restoration of flow may occur spontaneously, may be restored by administration of thrombolytic enzymes such as streptokinase or tissue plasminogen activator, or by surgical intervention or angioplasty.

The dosage of hemoglobin utilized in reperfusion therapy varies from patient to patient, but generally will fall in the range from 10 to 2500 mg/kg of body weight.
While hemoglobin acts to increase perfusion as indicated by increased blood flow, in some indications it does not appear to act by this mechanism in cardiac reperfusion. However, low doses in the range from 75 to 750 mg/kg of body weight are generally preferred and are efficacious pharmacologically. The dramatic limiting of reperfusion injury and consequent reduction of permanent cell damage in the area at risk cannot presently be fully explained, and Applicants therefore do not wish to be bound to any particular theory.

Ideally, a physician will administer an amount of hemoglobin which confers the desired effect of optimally suppressing reperfusion injury, thereby preserving tissue viability after blockage, and minimizing permanent cellular damage. Suppressing reperfusion injury has indirect benefits in addition to limiting tissue damage in the region at risk. For a review of reperfusion injury, refer to the book by Jennings and Yellon, Myocardial Protection: The Pathophysiology of Reperfusion and Reperfusion Injury (Raven Press, Ltd., N.Y. (1992) As shown in Example 1, use of hemoglobin results in a prolongation in the time at which cardiac arrhythmias arise, and lowers their frequency. Thus hemoglobin administration provides a method of reducing the frequency of ventricular arrhythmias, and in turn preventing cardiac arrest.

Another indirect benefit of hemoglobin administration is in preventing or reducing the incidence of restenosis. It is a not infrequent complication of angioplasty and surgical bypass techniques, that when the occlusion is relieved restenosis readily occurs. This may occur within minutes or hours after relief of the blockage, or at remote times of several weeks or longer. Thus, the unblocking procedures must be repeated, or the patient eventually succumbs because the same vessel whose blockage was relieved, has again become occluded. It is believed that residual damage to the vessel walls may attract cellular blood constituents, which adhere to the vessel lumen and initiate arteriosclerotic deposition. Surprisingly, after infusion of hemoglobin following relief of blockage statistically fewer vessels restenose than is observed in control groups. It is also observed that wall motion in the region adjacent to the infarct zone is dramatically improved in hemoglobin treated vs. HSA treated animals. Thus, the present invention provides a method for reducing restenosis in blood vessels from which occlusion has been relieved. This amount has been determined empirically as falling within 10 and 2500 mg/kg of body weight. As a practical matter, the physician can administer hemoglobin in increments until the mean arterial blood pressure has attained a value about 5 to 15 percent above the hemoglobin preadministration baseline. Applicants now understand that an increase in perfusion and the well-known pressor effect of hemoglobin are not necessarily causally linked, because suppression of the pressor effect by drugs such as prazosin does not impair the observed increase in perfusion.

The timing of administration should preferably be at a time just prior to relief of the blockage and reperfusion. Ideally this should be within 20 minutes of relief of blockage. However, treatment out to 1 hour prior to or after the relief of blockage may be beneficial, particularly when the blood vessel involved impacts a relatively small area at risk. In the case of cardiac blockage, a relatively small area at risk would involve about 5 to 25 percent of the myocardium.

The hemoglobin utilized in reperfusion therapy may be any type which has the following general properties: stroma-free, non-antigenic and non-pyrogenic (i.e. less than 0.25 endotoxin units per milliliter), and be free of bacterial and viral contamination. The hemoglobin may be isolated as disclosed in U.S. Patents 4,439,357, 4,526,715, 4,598,064, and 4,600,531. The hemoglobin is preferably rendered virus free, as disclosed in U.S. Patent No. 5,281,579.

The preferred hemoglobin is maintained in stable oxygen-releasing conformation by cross-linking. The best method of cross-linking involves a lysine-lysine bridge between the alpha subunits, as disclosed in U.S. Patents 4,600,531 and RE 34,271. Because the tetramer cannot fall apart, thereby retaining its 64,000 molecular weight, clearance from the blood stream is slowed. Further lengthening of blood retention time is effected by polymerizing the hemoglobin tetramers, as by polyamide linking groups. Alternative cross-linking and polymerizing techniques are described in Winslow, supra. One interesting technique involves simultaneous cross-linking and polymerizing with glutaraldehyde as disclosed in U.S. Patent No. 5,194,590.

Other advantages of the present invention will be apparent from the Examples, which follow.

### Example 1

An animal model system involving coronary occlusion was used to study the effect of hemoglobin perfusion therapy on controlling tissue damage resulting from sustained ischemia and reperfusion injury. The swine model is the model of choice because numerous studies have shown that the pig heart most closely resembles the human heart physiologically. For a review, see M. M. Swindle, ed., "Swine as Models in Biomedical Research", Iowa State University Press, (1992).

One particularly important criterion is the comparable absence in both the pig and humans of collateral flow. Collateral flow is the ability of the capillary bed of one arterial branch to compensate for an occlusion in another branch. The pig heart most closely resembles the human heart in showing a low degree of collateral flow capacity. See Bloor, et al., "The Pig as a Model of Myocardial Ischemia and Gradual Coronary Artery Occlusion", in Swine as Models in Biomedical Research, supra.

**Experimental Preparation.** Yorkshire swine of either sex (n=15), weighing 21.3 ± 1.4 kg, were initially sedated with Ketamine (10 mg/kg, i.m.) to allow placement of an intravenous catheter in an ear vein. Anesthesia was obtained with Pentobarbital Sodium (Nembutal®) 30 mg/kg, bolus i.v. injection, with a dose of 31.5 mg/hour given by continuous i.v. infusion, at a rate of 6.3 ml/hour (Sage Instruments Pump), to maintain a surgical plane of anesthesia. The swine were intubated and ventilated (Harvard Respirator). Respiratory status was monitored periodically with arterial blood gas determinations and ventilation rate and/or oxygen flow rate were adjusted to achieve physiological blood gas values. Bilateral femoral cutdowns were performed and the right femoral artery was cannulated with a 9F sheath (Cordis) and a 6F pigtail catheter was advanced under fluoroscopic guidance into the left ventricle. A right carotid cutdown was performed and the right carotid artery was cannulated with a 9F sheath. Three thousand units of Heparin sodium were administered intravenously and repeated doses of 1,000 units were given every 30 minutes. A bolus of 1 mg/kg of lidocaine was given i.v. and an infusion of 50 µg/kg/min was maintained throughout the experiment. Intravenous nitroglycerine was infused to achieve a 5-10 mmHg reduction in blood pressure during guide wire and balloon placement but was discontinued prior to balloon inflation. Electrocardiograph, blood pressure, and temperature monitoring was performed throughout the experiment.

A 7F AR2 guiding catheter (Scimed) was advanced to the left main coronary artery. Catheter position was confirmed and angiograms were performed using hand injections of 1-5 cc of iodinated contrast (Renografin-76®). A 0.014 inch Hi-Torque floppy guide wire (Advanced Cardiovascular Systems) was advanced into the first obtuse marginal branch of the circumflex coronary artery. A Hartzler ACX II® (2 mm diameter, 10 mm length) balloon angioplasty catheter (Advanced Cardiovascular Systems) was advanced over the guide wire into the first marginal branch. Care was taken to assure that the balloon did not obstruct flow in the main circumflex coronary artery. The balloon was inflated with just enough pressure to insure complete occlusion (2-4 ATM) of the first marginal branch for 90 minutes. Occlusion was confirmed by angiography.

**Study Protocol.** Prior to instrumentation the swine were randomized into one of two study groups. Ten minutes prior to balloon deflation the swine were intravenously infused at 5 ml/kg given over a five minute period (1 ml/kg/min) with either 10% diaspirin cross-linked hemoglobin (DCLHb™) or a Human Serum Albumin (HSA) solution which was oncotically matched to the hemoglobin solution (approximately 8% albumin). At ninety minutes the balloon was deflated and withdrawn. The animal was then allowed to reperfuse for 3 hours. An angiogram was performed after the 3 hour reperfusion period to document vessel patency. The animals were euthanized and the hearts rapidly removed.

**ECG Recording.** All pigs were instrumented with leads I, II, III, aVr, aVl, aVf, and the precordial lead V₄. The total number of arrhythmias were counted from the start of reperfusion to 45 minutes post-reperfusion. The time to onset of arrhythmias was measured from start of reperfusion to the onset of reperfusion arrhythmias. The total duration of the reperfusion arrhythmic period was calculated as the amount of time from the onset of reperfusion arrhythmias to a time point when the arrhythmias occurred less than one every 30 seconds. S-T segment changes following balloon occlusion were recorded from the isoelectric line either following the P or the T wave from the standardized precordial lead V₄.

**Myocardial blood flow.** Myocardial blood flow was measured using radioactive microspheres. Microspheres were injected at baseline, 60 minutes after occlusion, 5 minutes after the initiation of reperfusion and after 170 minutes of reperfusion. The radioactive microspheres were supplied as carbonized plastic spheres 15.5 ± 3.0 microns in diameter, which were labeled with either ¹⁵³Gd, ⁸⁵Sr, ⁴⁶Sc, or ¹¹³Sn.
The isotope is bonded into the carbonized plastic and does not leach from the sphere in saline or plasma. Microspheres (New England Nuclear) were obtained as 1 mCi of nuclide in 10 ml saline, to which 0.05% Tween-80®, a surface detergent, was added to minimize aggregation. Twenty µCi of the microspheres were removed from the sterile sealed vial with a syringe and diluted in saline to the appropriate concentration. The order of the microsphere injection was randomized to avoid bias of the data from microsphere lot or isotope type. The mixture of spheres was sonicated for at least 30 minutes prior to injection to assure complete dispersal. Immediately before injection, the microspheres were mechanically shaken with a Vortex® type mixer. Approximately 1.3 X 10⁶ microspheres were injected into the left ventricle and flushed with saline. In theory, the microspheres mix with the blood ejected from the left ventricle and are transported to the tissue in a similar pattern as red blood cells. The microspheres are trapped by the slightly smaller diameter capillaries (8 µ). The spheres remain lodged in the capillary bed with minimal migration until necropsy. To calibrate blood flow, an arterial blood flow sample was collected with a withdrawal rate of 2.06 ml/min during the time interval that the microspheres were infused. Following the determination of the areas at risk and the infarcted tissues, as described below, the left ventricular tissue slices were subdivided into epicardial, mid-myocardial, and endocardial thirds and the activity of each isotope was determined in a gamma counting system (Searl, Model 1185). Following this counting procedure, the tissue was divided into white, red and blue regions and recounted. The cardiac output and regional myocardial blood flow was calculated for each time point as previously described (Heyman, et al., "Blood flow measurement with radionuclide-labeled particles", Progress in Cardiovascular Disease, 20:55-79 (1977)).

**Analysis of myocardium at risk.** Immediately after the heart was removed, the first obtuse marginal branch of the circumflex coronary artery was isolated and cannulated. In addition, the left main coronary artery was cannulated to allow perfusion of both the left anterior descending and circumflex coronary arteries. Both vessels were simultaneously perfused at 120 mmHg. The marginal branch was perfused with 1.0% triphenyltetrazolium chloride (Sigma) and the left main coronary artery was perfused with 0.05% monastral blue. Triphenyltetrazolium chloride stains viable myocardium red and does not stain areas of necrotic or infarcted tissue. The heart was incubated in saline at 37°C for 20 minutes to allow staining. The heart was then perfusion fixed with formalin. The mean total weight of the left ventricle was comparable for the DCLHb and HSA groups, 55.3 ± 2.3 and 53.1 ± 5.2 grams, respectively.

The heart was sectioned into 0.5 cm thick transverse slices with a mechanical slicer and each slice was weighed. The basilar surface of each slice was photographed. Each photograph was scanned into a MacIntosh computer (Scanjet scanner, Adobe photoshop program) and, using a computer-aided planimetric program (NIH Image), the area at risk and the area of infarction were quantitated. The area of infarction was expressed as a percent of the area at risk.

**Data Analysis.** Data are presented as mean values ± SEM. Differences between groups at single time points were evaluated by the Student's *t*-test for unpaired data. For groups with significant disparities between standard deviations, nonparametric Mann-Whitney U-statistical analysis was performed. Differences among groups and between groups for multiple data points were compared by analysis of variance. The 0.05 level of significance was used to evaluate the statistical differences.

**Hemodynamic Data.** Heart rate and mean arterial blood pressure (MAP) remained constant during the first ninety five minutes of the experiment for both DCLHb and HSA treated groups (Table 1). Pigs receiving HSA showed a significant decrease in MAP at the 3 hour reperfusion period. Heart rate showed a significant 30% decrease from control in the DCLHb group at the 3 hour time point. Cardiac output was not significantly different from control during the occlusion or 5 minute reperfusion periods, but was significantly reduced in both the DCLHb and HSA groups at the 3 hour reperfusion period. Cardiac output was not different between the DCLHb and HSA groups at any time point. Calculated total peripheral resistance (TPR) was not significantly different between the DCLHb and HSA groups at either the control or occlusion time intervals. However, the DCLHb group had a significant increase in TPR at 5 minutes and 3 hours of reperfusion.

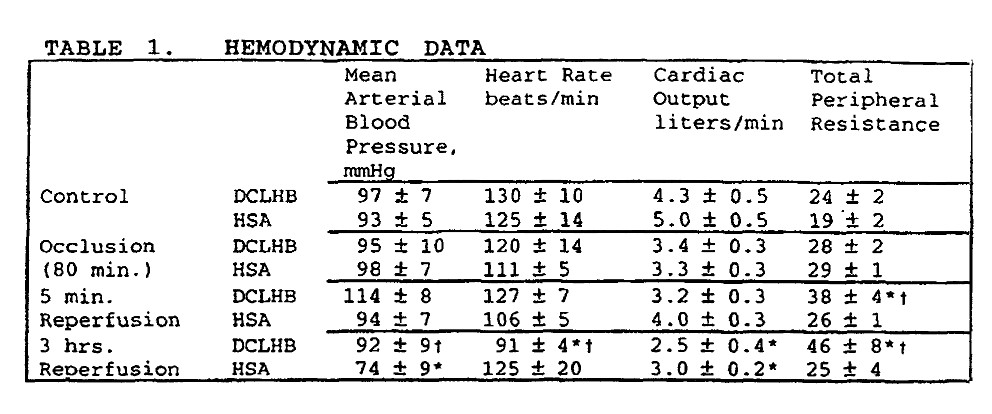

**Blood Data.** Table 2 shows that arterial pH was not significantly different between any of the time periods in either group. Arterial pH was 7.51 ± 0.01 with a range between 7.46 and 7.54. Although both pCO₂ and pO₂ remained very stable throughout the experiment, PCO₂ was significantly different from the HSA group at the 5 minute reperfusion sample interval in the DCLHb treated group. pO₂ in the DCLHb group was significantly increased above the HSA group at the occlusion time period.

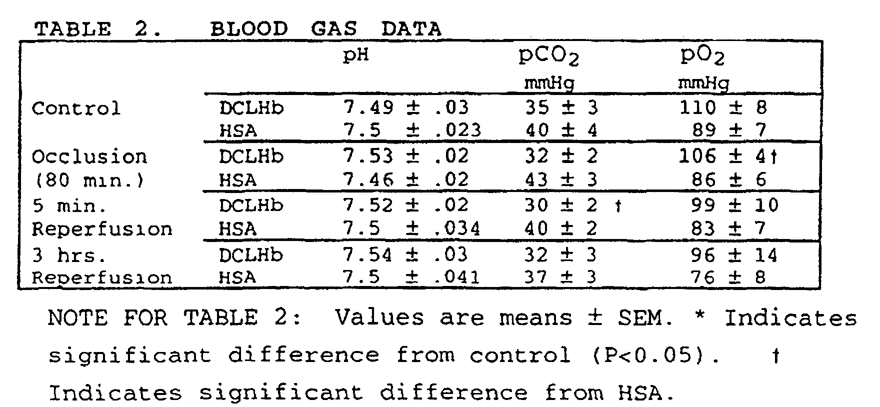

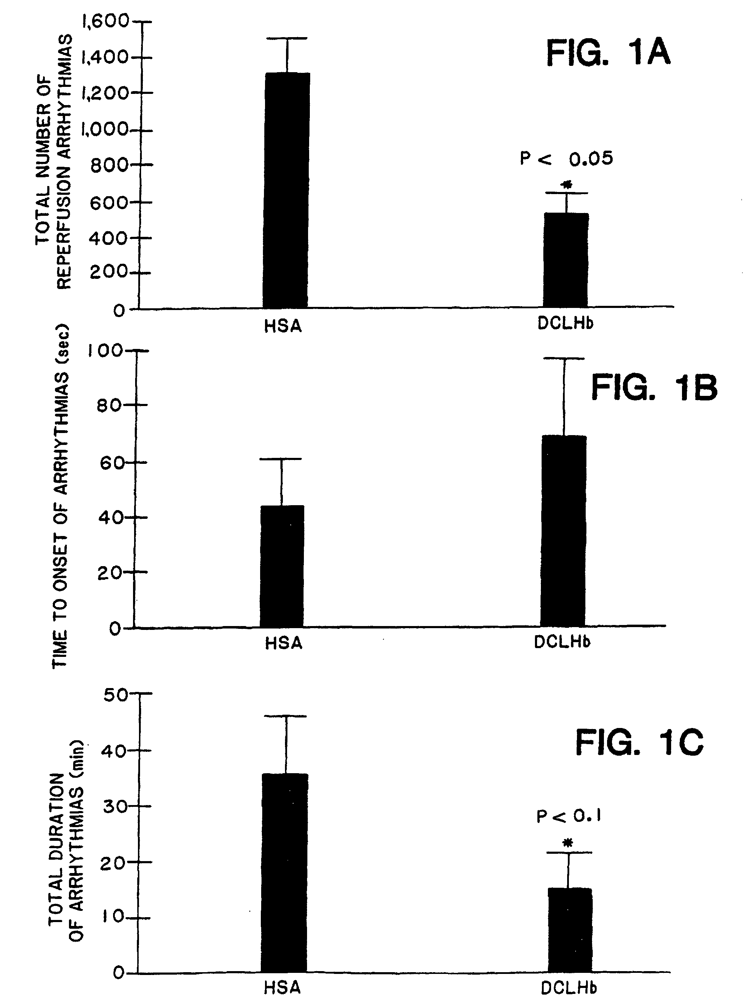

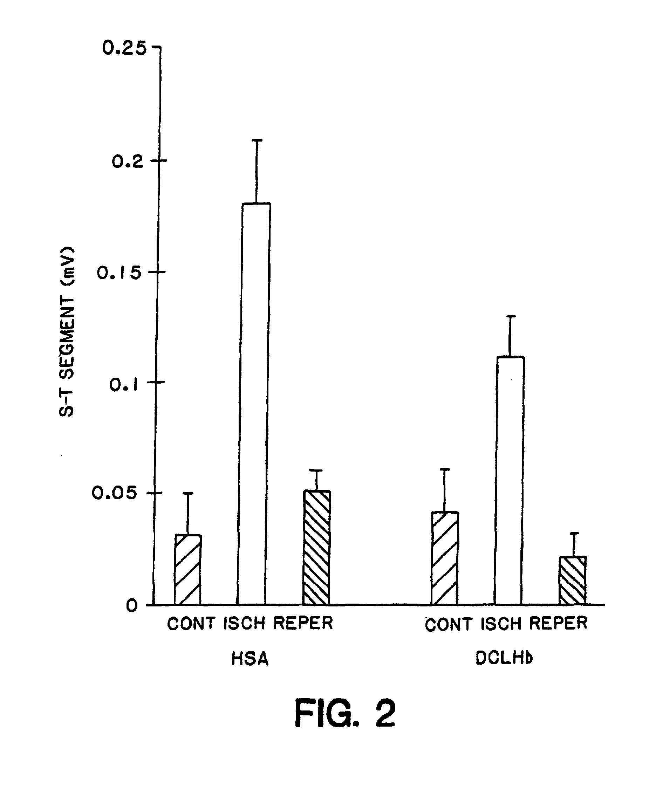

**ECG Data.** Reperfusion arrhythmias were noted in both the DCLHb and HSA groups (Figure 1); however, the total number of reperfusion arrhythmias, from start of reperfusion to 45 minutes post-reperfusion, was greater in the HSA group (1274 ± 222) than the DCLHb group (437 ± 198). The time to onset of arrhythmias (DCLHb, 67.5 ± 28.4 seconds; HSA, 43.7 ± 17.0 seconds) and the total duration of the arrhythmic period (DCLHb, 14.5 ± 6.5 minutes; HSA, 35.2 ± 10.9 minutes) were not statistically different for the two groups; however, there was a trend for DCLHb to increase the time to onset and to decrease the total duration of the arrhythmic period. Balloon occlusion produced a significant S-T segment elevation from control in both groups (DCLHb, 0.11 ± 0.02 mV; HSA, 0.18 ± 0.03 mV) (Figure 2). There was no statistical difference between the two groups with regard to S-T segment elevation during the occlusion or 3 hour reperfusion time period. DCLHb had reduced the S-T segment elevation to 0.02 ± 0.01 mV while the HSA treated animals still showed a 0.05 ± 0.01 mV S-T segment change.

Table 3 shows a comparison of DCLHb infused animals with those either infused with HSA or nothing (control). With respect to both the number of arrhythmias detected and the length of time to onset of arrhythmias, the control (no treatment) most favorably compared to the HSA treated group.

**TABLE 3.**

| **SWINE ISCHEMIA/REPERFUSION ECG DATA** | | | | |
|---|---|---|---|---|
| | | HSA | DCLHb | Control |
| Number of Arrhythmias | | 1274 ± 222 | 437 ± 198* | 1256 ± 434 |
| Duration of Arrhythmias (Minutes) | | 35 ± 11 | 14 ± 6 | 18 ± 6 |
| Time to Start of Arrhythmias (Seconds) | | 44 ± 17 | 67 ± 28 | 35 ± 15 |
| S-T | Baseline | 0.03 ± 0.02 | 0.04 ± 0.02 | 0.07 ± 0.01 |
| Segment | Ischemia | 0.18 ± 0.03 | 0.11 ± 0.02 | 0.36 ± 0.01* |
| Changes (mVolts) | Reperfusion | 0.05 ± 0.01 | 0.02 ± 0.01 | 0.02 ± 0.01 |

| | | | | |
|---|---|---|---|---|
| *Significantly different from other two treatment groups p<0.05. | | | | |

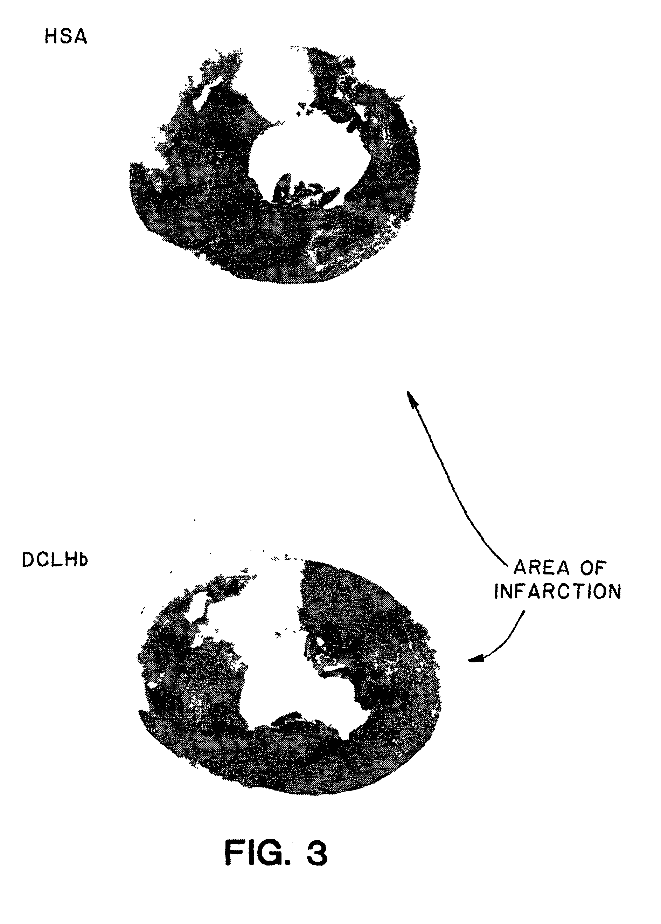

**Blood Flow Data.** Myocardial blood flow data are presented in Tables 4A and 4B. Table 4A shows blood flow to the epicardium, mid myocardium, endocardium and the endocardial:epicardial (endo/epi) blood flow ratio to an area of the free wall of the left ventricle that was not at risk for ischemia or infarction. We routinely examined tissue from the posterior wall of the left ventricle that was not perfused by the circumflex vessel. There were no differences in myocardial blood flow or endo/epi ratios with the exception of at the 3 hour reperfusion time interval in the DCLHb treated group, which exhibited a significant reduction in epicardial blood flow from control. Table 4B demonstrates the same parameters in tissue that is at risk for infarction. These blood flow measurements include tissue from both the ischemic area (white) and the area which was at risk but not ischemic (red). There was no significant difference between tissues of this region and the tissues in Table 4A during the control measurement period. The occlusion period produced a significant reduction in blood flow to all three regions of the myocardium in tissue at risk in pigs treated with either DCLHb or HSA. The endo/epi ratio was increased in both DCLHb and HSA groups during occlusion, indicating a proportionally greater reduction in blood flow to the epicardial region as compared to the endocardial layer of the myocardium. During the 5 minute reperfusion time period, there was a dramatic hyperemia to epi, mid , and endocardial tissue in both DCLHb and HSA groups with the exception of the endocardial region in the HSA group. The endo/epi ratio was therefore significantly less than that in the control group. Blood flows returned to control values at the 3 hour reperfusion period with the exception of the flow to the epicardial region in the DCLHb treated group. In this sample there was a significant difference in the pigs receiving DCLHb from control values and from the same tissue in the HSA treated group. Table 5 shows myocardial blood flow from the same hearts as included in Tables 4A and B, but this tissue has been divided into areas that were stained red (area at risk but not infarcted), tissues that were white (areas that did not take up the stain, therefore this area was infarcted) and the total combined flow to this region. Flow fell significantly to these regions during occlusion. The flow to the area at risk is defined as the collateral blood flow and was not significantly different between DCLHb and HSA. (See Figure 3). Since this flow was measured prior to treatment, these two flows should be similar. During occlusion the infarcted area showed tissue flows that were not significantly different from zero. The 5 minute reperfusion data demonstrate significant active hyperemia to all tissues in both DCLHb and HSA treated groups and there was no difference between flows in the two groups. At the 3 hour time point, blood flow to both the area at risk and the infarcted tissue in the DCLHb treated group was significantly reduced from control values while the corresponding flows in HSA treated animals had returned to control values.

**TABLE 4A.**

| **MYOCARDIAL BLOOD FLOW, ml/min/100g (Tissue Not in Area at Risk)** | | | | | |
|---|---|---|---|---|---|
| | | EPI | MID | ENDO | ENDO/EPI |
| Control | DCLHb | 171 ± 31 | 195 ± 33 | 212 ± 38 | 1.3 ± 0.2 |
| | HSA | 143 ± 17 | 167 ± 19 | 180 ± 18 | 1.3 ± 0.1 |
| Occlusion (80 min.) | DCLHb | 135 ± 23 | 143 ± 21 | 160 ± 16 | 1.3 ± 0.1 |
| | HSA | 117 ± 16 | 144 ± 20 | 154 ± 20 | 1.3 ± 0.1 |
| 5 min. Reperfusion | DCLHb | 147 ± 18 | 188 ± 26 | 200 ± 22 | 1.4 ± 0.1 |
| | HSA | 139 ± 17 | 165 ± 19 | 174 ± 21 | 1.3 ± 0.1 |
| 3 hrs. Reperfusion | DCLHb | 89 ± 15* | 113 ± 20 | 130 ± 17 | 1.5 ± 0.1 |
| | HSA | 105 ± 18 | 126 ± 24 | 142 ± 26 | 1.4 ± 0.1 |

**TABLE 4B.**

| **MYOCARDIAL BLOOD FLOW, ml/min/100g (Ischemic Tissue)** | | | | | |
|---|---|---|---|---|---|
| | | EPI | MID | ENDO | ENDO/EPI |
| Control | DCLHb | 181 ± 28 | 188 ± 30 | 217 ± 39 | 1.2 ± 0.1 |
| | HSA | 152 ± 19 | 176 ± 23 | 202 ± 24 | 1.3 ± 0.1 |
| Occlusion (80 min.) | DCLHb | 81 ± 15* | 92 ± 19* | 121 ± 20* | 1.6 ± 0.1* |
| | HSA | 60 ± 14* | 78 ± 17* | 117 ± 22* | 2.1 ± 0.2* |
| 5 min. Reperfusion | DCLHb | 325 ± 29* | 308 ± 24* | 312 ± 29* | 1.0 ± 0.1 |
| | HSA | 301 ± 33* | 292 ± 42* | 268 ± 35 | 0.9 ± 0.1* |
| 3 hrs. Reperfusion | DCLHb | 95 ± 15*† | 102 ± 19 | 119 ± 18 | 1.3 ± 0.1 |
| | HSA | 157 ± 36 | 168 ± 37 | 179 ± 35 | 1.2 ± 0.2 |
| NOTE FOR TABLE 4B: Values are means ± SEM. * Indicates significant difference from control (P<0.05). † Indicates significant difference from HSA (P<0.05). EPI = Epicardial tissue, MID = middle 1/3 of Myocardial tissue, ENDO = Endocardial tissue. ENDO/EPI represents the ratio of endocardial blood flow to epicardial blood flow. | | | | | |

**TABLE 5.**

| **MYOCARDIAL BLOOD FLOW (ml/min/100g) TO AREA AT RISK AND AREA INFARCTED** | | | | |
|---|---|---|---|---|
| | | Area at Risk | Area Infarcted | TOTAL |
| Control | DCLHb | 242.09 ± 49.04 | 178.64 ± 37.20 | 210.78 ± 40.68 |
| | HSA | 234.95 ± 34.23 | 176.15 ± 32.42 | 203.30 ± 34.96 |
| Occlusion (80 min.) | DCLHb | 86.02 ± 10.89* | 2.45 ± 0.80* | 57.21 ± 12.05* |
| | HSA | 79.62 ± 16.55* | 9.94 ± 3.18* | 55.54 ± 14.57* |
| 5 min. Reperfusion | DCLHb | 514.89 ± 90.85* | 390.79 ± 64.69* | 441.65 ± 70.75* |
| | HSA | 522.01 ± 71.64* | 313.84 ± 58.64* | 407.02 ± 64.50* |
| 3 hrs. Reperfusion | DCLHb | 121.29 ± 19.27*† | 102.86 ± 22.36* | 105.74 ± 13.93* |
| | HSA | 233.93 ± 49.06 | 166.00 ± 45.48 | 196.85 ± 48.27 |
| NOTE FOR TABLE 5: Values are means ± SEM. * Indicates significant difference from control (P<0.05). † Indicates significant difference from HSA. Column one "Area at Risk" is total collateral blood flow during occlusion. Column two "Area Infarcted" is the area of no flow only. Column three is the flow to the entire area of infarction plus area at risk. | | | | |

Table 6 demonstrates that in an anatomically paired organ, microspheres were equally distributed between the left and right kidney and that there was no significant difference between renal blood flow between the DCLHb and the HSA treated groups. These measurements are presented to validate the microsphere technique in this model.

**TABLE 6.**

| **KIDNEY BLOOD FLOW (ml/min/100g)** | | | | |
|---|---|---|---|---|
| | | LEFT | RIGHT | TOTAL |
| Control | DCLHb | 276 ± 53 | 292 ± 51 | 284 ± 51 |
| | HSA | 330 ± 27 | 315 ± 34 | 323 ± 30 |
| Occlusion (80 min.) | DCLHb | 265 ± 34 | 278 ± 35 | 271 ± 34 |
| | HSA | 299 ± 07 | 291 ± 13 | 295 ± 09 |
| 5 min. Reperfusion | DCLHb | 237 ± 28 | 253 ± 25 | 244 ± 26 |
| | HSA | 331 ± 30 | 334 ± 30 | 338 ± 26 |
| 3 hrs. Reperfusion | DCLHb | 206 ± 29 | 223 ± 33 | 214 ± 30 |
| | HSA | 324 ± 46 | 311 ± 53 | 317 ± 48 |
| NOTE FOR TABLE 6: Values are means ± SEM. None of the values listed are different from control nor are there differences between treatment groups. There are no differences between right and left kidney flows. LEFT = Left Kidney Flow, RIGHT = Right Kidney Flow, TOTAL = Total Kidney Flow. | | | | |

**Infarction Data.** Infarct size and areas at risk in DCLHb and HSA treated hearts are shown in Table 7. The percent of the total left ventricle that was at risk was 14.6 ± 2.6% for the DCLHb group and 10.6 ± 2.1% for the HSA group. These values were not significantly different. The total area at risk was 1126 ± 218 mm³ and 858 ± 173 mm³ for DCLHb and HSA treated groups respectively. The total infarcted area for DCLHb was 326 ± 91 mm³ and 456 ± 101 mm³ for HSA. These data then yield the percent of infarcted tissue as compared to the area at risk. In the DCLHb group 30.9 ± 6.1% of the area at risk was infarcted, while in the HSA group 53.2 ± 1.9% of the area at risk was infarcted. Since this is a ratio, the data were subjected to an Arcsine transformation for the statistical analysis. The DCLHb group was statistically different from the HSA group at P<0.009 using an unpaired t-test.

Intravenous infusion of DCLHb eighty minutes following occlusion of the first obtuse marginal branch of the circumflex coronary artery of the pig produced a significant reduction in the size of myocardial infarction compared to control animals which were infused with an oncotically matched human serum albumin solution. In addition, DCLHb significantly reduced detrimental reperfusion arrhythmias and produced a hemodynamically stable animal. Figure 1 shows transverse tissue sections through the swine myocardium. Comparison of the stained (vital) areas in the DCLHb and HSA perfused heart shows that the area of infarct is much smaller in the DCLHb perfused heart.

The myocardial blood flow data, as measured by radioactive microspheres, cannot account for the reduced infarction size in the DCLHb group. The only difference between the DCLHb and the HSA groups is the reduced epicardial blood flow and reduced blood flow to the area at risk in the DCLHb group at the 3 hour reperfusion time point. Reduction in flow at this time point should not correlate with an improvement in oxygen delivery and a reduction in infarction size.

**TABLE 7.**

| **INFARCTION DATA** | | | | |
|---|---|---|---|---|
| | % of LEFT VENTRICLE AT RISK | TOTAL AREA AT RISK mm³ | TOTAL AREA INFARCTED mm³ | % OF THE AREA AT RISK INFARCTED |
| DCLHb | 14.6 ± 2.6 | 1126 ± 218 | 326 ± 91 | 30.9 ± 6.1† |
| HSA | 10.6 ± 2.1 | 858 ± 173 | 456 ± 101 | 53.2 ± 1.9 |
| NOTE FOR TABLE 7: Values are means ± SEM. † Indicates significant difference from HSA (P<0.05). | | | | |

### Example 2

The general procedures described in the experiments of Example 1 were repeated in another series of swine; however, the procedures were carried out under conditions of asepsis, so that the wounds could be closed, and the animals revived. The pigs were allowed to convalesce for a period of 21 days, and were then sacrificed. Table 8 shows the effect of infusing DCLHb and human serum albumin respectively, on reperfusion immediately after surgery, and at 21 days. The data show that hemoglobin infusion is highly correlated with maintenance of reperfusion and the absence of restenosis. Thus, the present invention provides a method of maintaining reperfusion to remote times, thereby reducing the incidence of restenosis of blood vessels in which a prior occlusion was relieved.

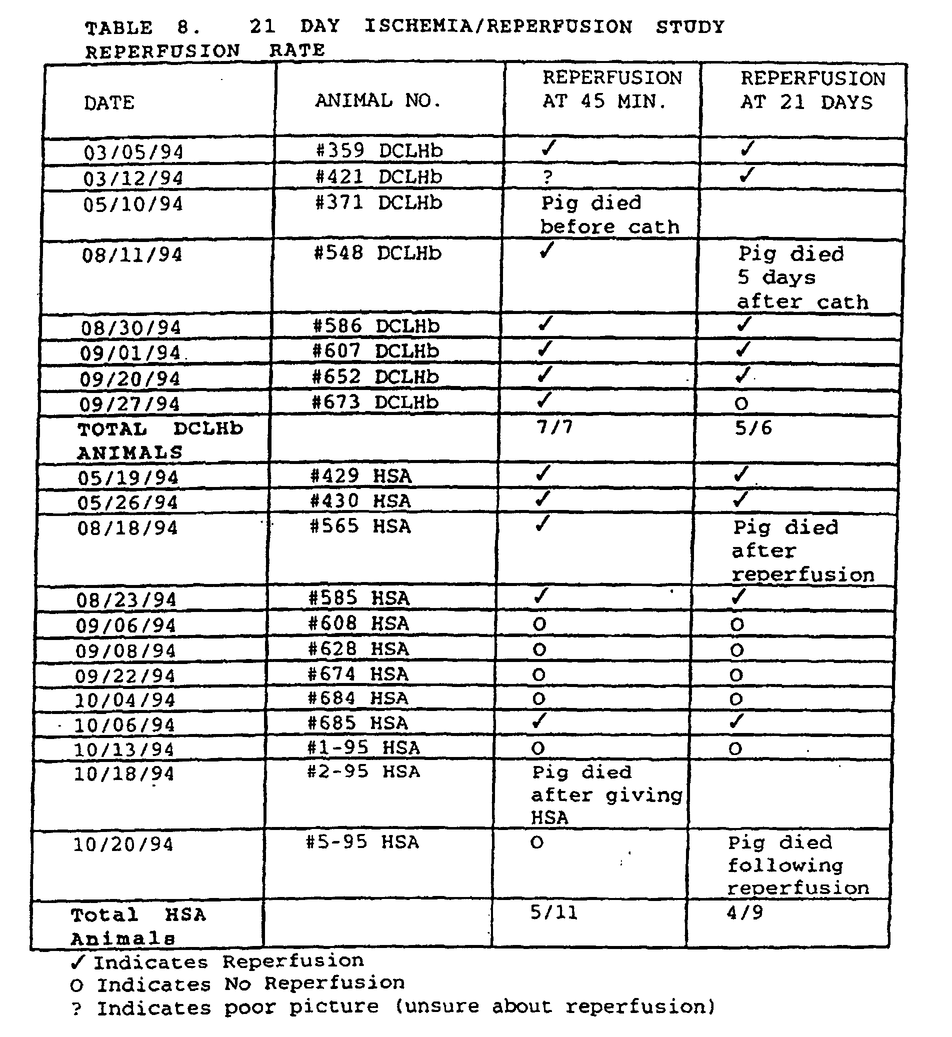

### Example 3

York swine of either sex, weighing 18.16 to 22.7 kg (40-50 lbs.), were surgically instrumented. Swine were initially sedated with Ketamine (10 mg/kg, i.m.) to allow placement of an intravenous catheter in the ear vein. Anesthesia was induced with sodium pentothal (10 mg/kg, i.v.). The trachea was intubated with a 6 or 7 mm endotracheal, and the animal was ventilated with a Harvard respirator and atelectasis was prevented with 3-5 cm H₂O positive end expiratory pressure. A surgical plane of anesthesia was maintained with an infusion of sodium pentothal (1.2 mg/min). Mean arterial blood pressure and ECG were monitored continuously throughout the experiment. Under fluoroscopy, cardiac catheters were advanced to the appropriate locations. A 5F Pigtail catheter was advanced from the right femoral artery to the left ventricle. A control set of data was collected to include hemodynamic variables, blood gases, and cardiac output. A 9F sheath (Cordis) was placed in the left carotid artery and a 7F AR 2 guiding catheter was advanced to the left main coronary artery. A 0.014 inch floppy tip guide wire was advanced to the first obtuse marginal branch of the circumflex coronary artery and a Hartzler ACX coronary dilation catheter was advanced over the guide to a point just distal to the main circumflex artery. The coronary balloon catheter was inflated and remained inflated for 90 minutes. At 80 minutes of balloon inflation either DCLHb or HSA, 5 ml/kg, was infused at a rate of 1 ml/min/kg for five minutes. The balloon was deflated and the animal was observed for forty-five minutes of reperfusion. At this time the final data set was collected, and the pig was recovered from anesthesia.

Twenty-one days after the occlusion the pig was anesthetized in the same method as indicated above, and a coronary angiogram and a left ventriculogram was recorded in the 60° LAO position. Using a MedRad injector 25-40 ml of iodinated contrast (Renografin-76) was infused into the left ventricle at a flow rate of 15 ml/min. The heart was rapidly removed, the right ventricle trimmed off and the left ventricle sliced in 5 mm wide rings from the apex to the base, and each ring was placed into 4% phosphate buffered formaldehyde. Hearts were sent for microscopic analysis.

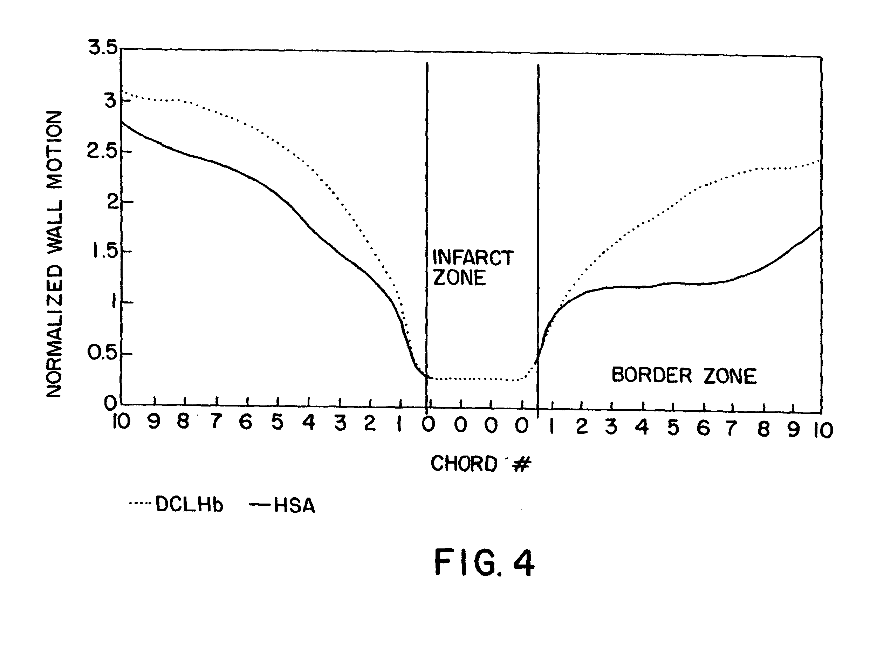

The ventriculogram was analyzed using centerline analysis, Sheehan et al., Circulation, 74(2)293 (1986). Wall motion was measured along 100 chords constructed perpendicular to a centerline drawn midway between the end-diastolic and end-systolic contours, normalized for heart size and plotted as relative wall motion index units. Chords were compared to the region of infarction, akinetic and to the area of normal wall motion, eukinetic. We specifically focused on the border region between eukinetic and akinetic, which is defined as hypokinetic. The regions of akinesis and hypokinesis were compared to the infarct related artery in the coronary angiogram. The results are shown in Figure 4, and show clearly an improvement in wall motion score of at least 0.15 relative index units in between the infarct zone and 20 chords in the tissue region at risk.

## Claims

1. Use of haemoglobin in the manufacture of a medicament for intravascular administration to provide systemic delivery and for preserving tissue viability after blockage of a blood vessel in a patient undergoing ischemia caused by coronary thrombosis.

2. Use of haemoglobin according to Claim 1, wherein the intravascular administration is by means of intravenous infusion.

3. Use of haemoglobin in the manufacture of a medicament for suppressing reperfusion injury to tissue whose nourishment has been disrupted by blockage of a blood vessel.

4. Use of haemoglobin in the manufacture of a medicament for reducing the frequency and duration to onset of cardiac arrhythmias following relief of arterial blockage in a patient.

5. Use of haemoglobin in the manufacture of a medicament for reducing the incidence of restenois of a blood vessel after relief of a blockage thereof.

6. Use of Claim 5 wherein the blockage is relieved by angioplasty.

7. Use of Claim 5 wherein the blockage is relieved by bypass surgery.

8. Use according to any one of Claims 1, 5, 6 or 7 wherein said medicament contains 10 to 2500 mg of haemoglobin per kg of body weight of the intended patient.

9. Use of haemoglobin in the manufacture of a medicament for improving contractile function in ischemic cardiac tissue following relief of heart vessel blockage.

10. Use according to Claim 9, wherein the medicament contains haemoglobin in an amount sufficient to provide a wall motion score improvement of at least 0.15 index units in the region between the infarct zone and 20 chords in the tissue region at risk.

11. Use according to any preceding claim, wherein the haemoglobin is crosslinked .

12. Use according to Claim 11, wherein the haemoglobin is diaspirin crosslinked.

13. Use according to Claim 11 , wherein the haemoglobin is both crosslinked and polymerised.

14. Use according to any one of the preceding claims wherein the patient is administered with 70 to 750mg of haemoglobin per kg of body weight of the patient.

15. Use according to any one of the preceding claims wherein the patient is human.

16. Use according to any one of the preceding claims wherein the medicament is administered up to 1 hour after relief of the blockage.

17. Use according to any one of claims 1 to 15 wherein the medicament is administered up to 1 hour before relief of the blockage.

18. Use according to claim 17 wherein the medicament is administered within 20 minutes before relief of the blockage.

19. Use according to any one of the preceding claims wherein the haemoglobin is stroma-free.

## Patentansprüche

1. Verwendung von Hämoglobin bei der Herstellung eines Medikaments zur intravaskulären Verabreichung zum Ermöglichen von systemischer Abgabe und zum Aufrechterhalten der Lebensfähigkeit von Gewebe nach einer Blockade eines Blutgefäßes bei einem Patienten, der unter durch Koronarthrombose verursachter Ischämie leidet.

2. Verwendung von Hämoglobin nach Anspruch 1, wobei die intravaskulare Verabreichung durch intravenöse Infusion erfolgt.

3. Verwendung von Hämoglobin bei der Herstellung eines Medikaments zum Unterdrücken einer Reperfusionsverletzung von Gewebe, dessen Erfährung durch Blockade eines Blutgefäßes unterbrochen worden ist.

4. Verwendung von Hämoglobin bei der Herstellung eines Medikaments zum Verringern der Frequenz und Dauer bis zum Einsetzen von Herzarrhythmien im Anschluß an die Aufhebung einer arteriellen Blockade bei einem Patienten.

5. Verwendung von Hämoglobin bei der Herstellung eines Medikaments zum Verringern der Häufigkeit einer Restenose eines Blutgefäßes nach Aufhebung einer Blockade desselben.

6. Verwendung nach Anspruch 5, wobei die Blockade durch Angloplastie aufgehoben wird.

7. Verwendung nach Anspruch 5, wobei die Blockade durch Bypassoperation aufgehoben wird.

8. Verwendung nach einem der Ansprüche 1, 5, 6 oder 7, wobei das Medikament 10 bis 2500 mg Hämoglobin/kg Körpergewicht des vorgesehenen Patienten enthält.

9. Verwendung von Hämoglobin bei der Herstellung eines Medikaments zum Verbessern der kontraktilen Funktion in ischämischem Herzgewebe im Anschluß an die Aufhebung einer Herzgefäßblockade.

10. Verwendung nach Anspruch 9, wobei das Medikament Hämoglobin in einer Menge enthält, die ausreicht, um eine Verbesserung des Wandbewegungs Scores von mindestens 0,15 Indexeinheiten in dem Bereich zwischen der Infarktzone und 20 Chordae in dem gefährdeten Gewebebereich zu ermöglichen.

11. Verwendung nach einem der vorhergehenden Ansprüche, wobei, das Hämoglobin vernetzt ist.

12. Verwendung nach Anspruch 11, wobei das Hämoglobin Diaspitin-vernetzt ist.

13. Verwendung nach Anspruch 11, wobel das Hämoglobin sowohl vernetzt als auch polymerisiert ist.

14. Verwendung nach einem der vorhergehenden Ansprüche, wobei dem Patienten 70 bis 750 mg Hämoglobin/kg Körpergewicht des Patienten verabreicht werden.

15. Verwendung nach einem der vorhergehen den Ansprüche, wobei der Patient ein Mensch ist.

16. Verwendung nach einem der vorhergehenden Ansprüche, wobei das Medikament bis zu 1 h nach Aufhebung der Blockade verabreicht wird.

17. Verwendung nach einem der Ansprüche 1 bis 15, wobei das Medikament bis zu 1 h vor Aufhebung der Blockade verabreicht wird.

18. Verwendung nach Anspruch 17, wobei das Medikament innerhalb von 20 min vor Aufhebung der Blockade verabreicht wird.

19. Verwendung nach einem der vorhergehenden Ansprüche, wobei das Hämoglobin stromafrei ist.

## Revendications

1. Utilisation d'hémoglobine dans la fabrication d'un médicament pour administration intravasculaire en vue de fournir un apport systémique et pour préserver la viabilité tissulaire après obstruction d'un vaisseau sanguin chez un patient atteint d'ischémie due à une thrombose coronaire..

2. Utilisation d'hémoglobine suivant la revendication 1, dans laquelle l'administration intravasculaire est effectuée au moyen d'une perfusion intraveineuse.

3. Utilisation d'hémoglobine dans la fabrication d'un médicament pour supprimer la lésion de reperfusion d'un tissu dont l'alimentation a été interrompue par obstruction d'un vaisseau sanguin.

4. Utilisation d'hémoglobine dans la fabrication d'un médicament pour réduire la fréquence et la durée des arythmies cardiaques initiales après soulagement de l'obstruction artérielle chez un patient.

5. Utilisation d'hémoglobine dans la fabrication d'un médicament pour réduire l'incidence de resténose d'un vaisseau sanguin après soulagement de son obstruction.

6. Utilisation suivant la revendication 5, dans laquelle l'obstruction est soulagée par angioplastie.

7. Utilisation suivant la revendication 5, dans laquelle l'obstruction est soulagée par pontage chirurgical.

8. Utilisation suivant l'une quelconque des revendications 1, 5, 6 ou 7, dans laquelle ledit médicament contient 10 à 2500 mg d'hémoglobine par kg de poids corporel du patient concerné.

9. Utilisation d'hémoglobine dans la fabrication d'un médicament pour améliorer la fonction contractile du tissu cardiaque ischémique après soulagement de l'obstruction d'un vaisseau du coeur.

10. Utilisation suivant la revendication 9, dans laquelle le médicament contient de l'hémoglobine selon une quantité suffisante pour fournir une amélioration de score de mouvement de paroi (en anglais : "wall motion score") d'au moins 0,15 unité dans la région entre la zone d'infarctus et 20 cordons (en anglais : "chords") dans la région tissulaire à risque.

11. Utilisation suivant l'une quelconque des revendications précédentes, dans laquelle l'hémoglobine est réticulée.

12. Utilisation suivant la revendication 11, dans laquelle l'hémoglobine est réticulée au moyen de diaspirine.

13. Utilisation suivant la revendication 11, dans laquelle l'hémoglobine est à la fois réticulée et polymérisée.

14. Utilisation suivant l'une quelconque des revendications précédentes, dans laquelle on administre au patient 70 à 750 mg d'hémoglobine par kg de poids corporel dudit patient.

15. Utilisation suivant l'une quelconque des revendications précédentes, dans laquelle le patient est un être humain.

16. Utilisation suivant l'une quelconque des revendications précédentes, dans laquelle le médicament est administré au plus tard 1 heure après le soulagement de l'obstruction.

17. Utilisation suivant l'une quelconque des revendications 1 à 15, dans laquelle le médicament est administré au plus tôt 1 heure avant le soulagement de l'obstruction.

18. Utilisation suivant la revendication 17, dans laquelle le médicament est administré dans les 20 minutes qui précèdent le soulagement de l'obstruction.

19. Utilisation suivant l'une quelconque des revendications précédentes, dans laquelle l'hémoglobine est dépourvue de stroma.
